(19) Europäisches Patentamt
European Patent Office
Office européen des brevets

(11) Veröffentlichungsnummer: **0 283 979 B1**

(12) **EUROPÄISCHE PATENTSCHRIFT**

(45) Veröffentlichungstag der Patentschrift: **09.09.92**

(51) Int. Cl.[5]: **C07C 403/08**, C07C 35/18, C07C 43/196, C07F 9/54

(21) Anmeldenummer: **88104437.4**

(22) Anmeldetag: **19.03.88**

# (54) Aethinylcyclohexenderivate.

(30) Priorität: **27.03.87 CH 1191/87**

(43) Veröffentlichungstag der Anmeldung:
**28.09.88 Patentblatt 88/39**

(45) Bekanntmachung des Hinweises auf die Patenterteilung:
**09.09.92 Patentblatt 92/37**

(84) Benannte Vertragsstaaten:
**AT BE CH DE FR GB IT LI NL**

(56) Entgegenhaltungen:
**EP-A- 0 004 621**
**AT-B- 367 011**
**DE-A- 2 037 935**
**DE-B- 1 046 612**

**JOURNAL OF THE CHEMICAL SOCIETY(C), 1971, D. E. LOEBER et al, "Carotenoids and Related Compounds. Part XXVIII. Synthesis of Zeaxanthin, Beta-Cryptoxanthin, and Zeinoxanthin (Alpha-Cryptoxanthin)", Seiten 404-408**

**PURE AND APPLIED CHEMISTRY, Band 51, 1979, H. MAYER, "Synthesis of optically active Carotenoids and Related Compounds", Seiten 535-564**

**CHEMICAL ABSTRACTS, Band 94, Nr. 11, 16. März 1981, Columbus, Ohio, USA; N. KAN et al, "Synthesis of 4-chlorovinylidene 3,3,5-trimethylcyclohexyl acetate", Seite 682,Spalte 1, Zusammenfassung Nr. 83 654f**

(73) Patentinhaber: **F. HOFFMANN-LA ROCHE AG**
**Postfach 3255**
**CH-4002 Basel(CH)**

(72) Erfinder: **Lukàc, Teodor**
**Birkenweg 1**
**CH-4147 Aesch(CH)**
Erfinder: **Soukup, Milan, Dr.**
**Blumenweg**
**CH-4332 Stein(CH)**

(74) Vertreter: **Cottong, Norbert A. et al**
**Grenzacherstrasse 124 Postfach 3255**
**CH-4002 Basel(CH)**

EP 0 283 979 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von Zeaxanthin und von Zwischenprodukten in der Zeaxanthinsynthese sowie neue Aethinylcyclohexenderivate in diesem Verfahren.

In der Literatur wurden bereits verschiedene Verfahren zur Herstellung von Zeaxanthin ausgehend von Isophoron, Ketoisophoron oder dessen Derivaten beschrieben. Die bekannten Verfahren haben jedoch gewisse Nachteile, wie grosse Gesamtstufenzahl oder problematische Einzelstufen, und ergeben unbefriedigende Gesamtausbeuten.

Die europäische Patentpublikation 120.341 beschreibt ein Verfahren zur Herstellung von Zwischenprodukten in Carotinoidsynthesen, nämlich zur Herstellung von 3-Methyl-2,4-pentadien-1-ol-Derivaten, und zwar dadurch, dass man ein cyclisches Keton, wie beispielsweise das Ketoisophoronderivat 4-(1-Methoxy-1-methyläthoxy)-2,2,6-trimethylcyclohexan-1-on, mit einem Lithiumsalz $LiC{\equiv}C{-}C(CH_3){\equiv}CH{-}CH_2{-}OR'$, worin OR' eine Aethergruppe darstellt, alkinyliert, das erhaltene Acetylenderivat mit einem Aluminiumhydrid reduziert und anschliessend hydrolysiert, und die so erhaltene Verbindung, wie beispielsweise 5-(1,4-Dihydroxy-2,2,6-trimethylcyclohexyl)-3-methyl-2,4-pentadien-1-ol, dehydratisiert.

Die europäische Patentpublikation 100.839 beschreibt ein Verfahren zur Herstellung von Phosphoniumsalzen von Polyenen, welches als Zwischenstufe in Carotinoid- und Retinoidsynthesen (z.B. von Zeaxanthin) geeignet ist. Die Phosphoniumsalze, z.B. [5-(4-Hydroxy-2,6,6-trimethyl-1-cyclohexen-1-yl)-3-methyl-2,4-pentadienyl]-triphenylphosphonium-chlorid, werden durch katalytische Hydrierung der entsprechenden acetylenischen Phosphoniumsalze hergestellt.

Die europäische Patentpublikation 4621 beschreibt u.a. die Herstellung von Zeaxanthin, die aus dem 2,2,6-Trimethyl-1,4-cyclohexandion ausgeht. In dem dort beschriebenen Verfahren wird dieses Ketoisophoronderivat in nicht weniger als zwölf Verfahrensstufen in Zeaxanthin übergeführt.

Auch die deutsche Offenlegungsschrift 2.037.935 beschreibt u.a. die Herstellung von Zeaxanthin, und zwar unmittelbar aus einem [5-(4-Hydroxy-2,6,6-trimethyl-1-cyclohexenyl)-3-methyl-2,4-pentadienyl]-triphenylphosphoniumhalogenid und 2,7-Dimethyl-octa-2,4,6-trien-1,8-dial. Das Phosphoniumsalz selbst wird aus 4-(4-Acetoxy-2,6,6-trimethyl-1-cyclohex-1-en-1-yl)-2-acetoxy-but-3-in in drei oder vier Verfahrensstufen über 3-Hydroxy-β-ionon (und eventuell auch noch 3-Hydroxy-ionon) und 5-(4-Hydroxy-2,6,6-trimethyl-1-cyclohexen-1-yl)-3-methyl-1,4-pentadien-3-ol hergestellt.

J. Chem. Soc. (C), 1971, 404-408, beschreibt u.a. die Herstellung von Zeaxanthin aus dem Ketiosophoronderivat 4-Aethylendioxy-2,6,6-trimethylcyclohexanon über u.a. 1-(4-Hydroxy-2,6,6-trimethyl-1-cyclohexen-1-yl)-3-methyl-1,4-pentadien-3-ol in neun Verfahrensstufen.

Pure and Applied Chemistry 51, 535-564 (1979) (eine Uebersicht über die Carotinoid-Chemie) beschreibt u.a. die Reduktion von 3-Hydroxy-β-ionol zum 3-Hydroxy-β-ionon, dessen Umwandlung mittels einer Grignard-Reaktion zum 1-[4-Hydroxy-2,6,6-trimethyl-1-cyclohexen-1-yl)-3-methyl-1,4-pentadien-3-ol, die Ueberführung dieses Alkohols in das [5-(4-Hydroxy-2,6,6-trimethyl-1-cyclohexen-1-yl)-3-methyl-2,4-pentadienyl]triphenylphosphoniumchlorid oder -bromid und schliesslich die Umsetzung des Phosphoniumsalzes mit 2,7-Dimethyl-octa-2,4,6-trien-1,8-dial zum Zeaxanthin.

Das erfindungsgemässe Verfahren zur Herstellung von Zeaxanthin und von Zeaxanthin-Zwischenprodukten ist dadurch gekennzeichnet, dass man eine Verbindung der allgemeinen Formel

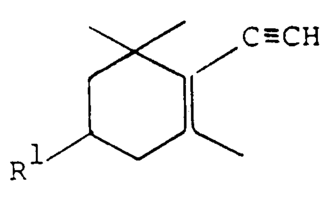

I

worin $R^1$ Hydroxy oder eine verätherte Hydroxygruppe bedeutet,

in einem inerten organischen Lösungsmittel in das Acetylenid überführt und dieses mit Methylvinylketon umsetzt, das erhaltene Alkoholat oder den nach Hydrolyse des Alkoholates erhaltenen Alkohol der allgemeinen Formel

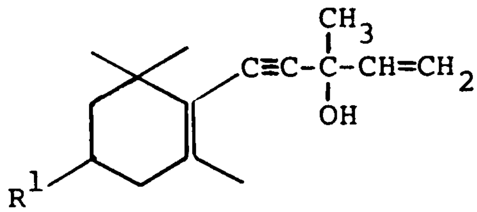

II

worin $R^1$ die obige Bedeutung hat,
in einem inerten organischen Lösungsmittel mit einem Aluminiumhydrid der allgemeinen Formel

$R_xMAlH_{4-x}$ III

worin M Alkalimetall bedeutet, R $C_1$-$C_{10}$-Alkoxy oder eine Gruppe der Formel $C_nH_{2n+1}$-O-$C_mH_{2m}$-O- darstellt, m und n unabhängig voneinander ganze Zahlen von 1 bis 7 bezeichnen und x für 0, 1, 2 oder 3 steht,
reduziert und anschliessend zur Verbindung der allgemeinen Formel

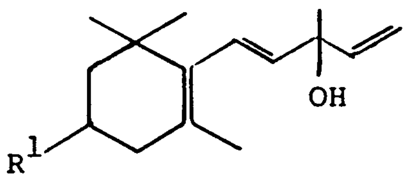

IV

worin $R^1$ die obige Bedeutung hat,
hydrolysiert und dass man, gewünschtenfalls, die erhaltene Verbindung der Formel IV in Zeaxanthin überführt.

Gemäss dem vorliegenden Verfahren erfolgt die Herstellung der Verbindungen der Formel IV durch stufenweise Einführung der 3-Hydroxy-3-methyl-1,4-pentadienylgruppe via Verbindungen der Formel I und Umsetzung mit einem $\alpha,\beta$-ungesättigten Keton zu Verbindungen der Formel II bzw. deren Alkoholaten. Ueberraschenderweise wird auf diesem Wege eine beträchtliche Verbesserung der Gesamtausbeute und eine Vereinfachung des Gesamtverfahrens erzielt. Alle Stufen des Verfahrens, einschliesslich der Herstellung der Verbindungen der Formel I, können leicht im technischen Massstab durchgeführt werden. Ferner können aufwendige Reinigungsoperationen vermieden werden. Wird beispielsweise die Verbindung der Formel IV via Phosphoniumsalz zu Zeaxanthin weiter umgesetzt so kann auf eine Reinigung der Verbindungen der Formeln I, II und IV verzichtet werden und lediglich eine Umkristallisation des Phosphoniumsalzes erfolgen. Gewünschtenfalls kann auch die Hydroxyverbindung der Formel I leicht durch Umkristallisation gereinigt werden.

Das erfindungsgemässe Verfahren eignet sich insbesondere auch zur Herstellung von natürlichem (3R, 3'R)-Zeaxanthin. Die Umsetzung der Verbindungen der Formel I zu den Verbindungen der Formel IV einschliesslich der Einführung verätherter Hydroxygruppen $R^1$ kann vorzugsweise auch als Eintopfverfahren durchgeführt werden.

Der Ausdruck "verätherte Hydroxygruppe" umfasst im Rahmen der vorliegenden Erfindung übliche Aetherschutzgruppen, beispielsweise Alkoxy wie Methoxy, Aethoxy, t-Butoxy oder Isobutoxy, Arylalkoxy wie Benzyloxy, Trialkylsilyloxy wie Trimethylsilyloxy, oder Gruppen der allgemeinen Formel

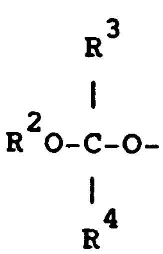

V

worin $R^2$ Alkyl bedeutet und $R^3$ und $R^4$ unabhängig voneinander Wasserstoff oder Alkyl bedeuten, oder $R^2$ und $R^3$ zusammen auch Tetramethylen bedeuten.
Vorzugsweise steht in den genannten Gruppen Alkyl für $C_1$-$C_7$-Alkyl, Alkoxy für $C_1$-$C_7$-Alkoxy und Aryl für Phenyl. Bevorzugte verätherte Hydroxygruppen sind Trialkylsilyloxy und die Gruppen der Formel V, wie Trimethylsilyloxy, 1-Methoxy-1-methyläthoxy, Tetrahydropyranyloxy und dergleichen.

Der Ausdruck "Alkalimetall" umfasst Lithium, Natrium und Kalium. Der Ausdruck "Halogen" umfasst Fluor, Chlor, Brom und Jod, vorzugweise Chlor und Brom. Der Ausdruck "Alkyl" umfasst insbesondere $C_1$-$C_7$-Alkyl, wie Methyl, Aethyl oder Butyl. Der Ausdruck "Aryl" umfasst insbesondere carbocyclische Gruppen, wie Phenyl, Tolyl und dergleichen, insbesondere Phenyl. Der Ausdruck "Acyloxy" bezeichnet übliche Esterschutzgruppen, wie Benzoyloxy und $C_1$-$C_7$-Alkanoyloxy, insbesondere Acetoxy.

Die Umsetzung einer Verbindung der Formel I mit Methylvinylketon zu einem Alkohol der Formel II oder dessen Alkoholat kann in an sich bekannter Weise in einem inerten organischen Lösungsmittel erfolgen. Beispiele geeigneter Lösungsmittel sind die Aether und die gesättigten oder aromatischen Kohlenwasserstoffe, wie Tetrahydrofuran, Dioxan, Diäthyläther, Petroläther, Hexan, Benzol, Toluol, Xylol und dergleichen. Bevorzugte Lösungsmittel sind die Aether, insbesondere Tetrahydrofuran. Die Umsetzung kann mit einer Verbindung der Formel I erfolgen, worin $R^1$ Hydroxy bedeutet. Vorzugsweise wird jedoch eine Verbindung der Formel I umgesetzt, worin $R^1$ eine verätherte Hydroxygruppe, insbesondere Trialkylsilyloxy oder eine Gruppe der Formel V bedeutet.

Die Deprotonierung der Verbindungen der Formel I kann mit üblichen zur Deprotenierung von Acetylenen geeigneten Basen, insbesondere mit Lithium-, Natrium- oder Magnesiumbasen erfolgen. Beispiele geeigneter Basen sind lithiumorganische Verbindungen, wie Methyllithium, Butyllithium oder Phenyllithium, Grignard-Reagentien, wie Alkylmagnesiumhalogenide und Dialkylmagnesium, Amide, wie Lithiumamid und Natriumamid, Hydride, wie Lithiumhydrid und Natriumhydrid, und dergleichen. Bevorzugte Basen sind Alkyllithium und Alkylmagnesiumbromid. Vorzugsweise wird ein leichter Ueberschuss an Base bezogen auf die Verbindung der Formel I verwendet, beispielsweise etwa 1,1-1,3 Aequivalente.

Das Methylvinylketon wird vorzugsweise im Ueberschuss bezogen auf die Verbindung der Formel I verwendet. Vorzugsweise werden mindestens etwa 1,3 Moläquivalente Methylvinylketon eingesetzt. Im allgemeinen ist eine Menge von etwa 1,4-2,0 Moläquivalenten, insbesondere etwa 1,5-1,8 Moläquivalenten bevorzugt.

Die Umsetzung mit Methylvinylketon erfolgt vorzugsweise in Gegenwart eines anorganischen Lithium- oder Cersalzes. Hierbei sollte darauf geachtet werden, dass das Salz im verwendeten Lösungsmittel ausreichend löslich ist. Beispiele geeigneter Salze sind Lithiumhalogenid, Cerhalogenid, Lithium-tetrafluoroborat und dergleichen. Bevorzugt sind Certrichlorid und insbesondere Lithiumbromid. Die Menge an Lithium- oder Cersalz ist nicht kritisch und kann beispielsweise etwa 0,5-2,0 Aequivalente oder weniger bezogen auf die Verbindung der Formel I betragen.

Temperatur und Druck bei der Umsetzung einer Verbindung der Formel I zum Alkoholat der Verbindung der Formel II sind nicht kritisch. Im allgemeinen wird jedoch bei Atmosphärendruck und Raumtemperatur oder tieferer Temperatur, beispielsweise bis etwa -30 °C, gearbeitet. Besonders bevorzugt ist ein Temperaturbereich von etwa -20 °C bis etwa 0 °C. Entsprechend den bevorzugt verwendeten Basen wird vorzugsweise das Lithium-, Natrium- oder Magnesiumalkoholat der Verbindung der Formel II erhalten.

Gewünschtenfalls kann das erhaltene Alkoholat zum Alkohol der Formel II hydrolysiert werden. Die Hydrolyse kann nach den für die Hydrolyse von Alkoholaten üblichen Methoden erfolgen, beispielsweise mit Wasser. Vorzugsweise wird jedoch das Alkoholat nicht hydrolysiert, sondern direkt zur Verbindung der Formel IV weiter umgesetzt, da auf diesem Wege weniger Reduktionsmittel verbraucht wird.

Die Verbindung der Formel II oder dessen Alkoholat kann in an sich bekannter Weise mit einem Aluminiumhydrid der Formel III reduziert werden. Hierbei wird im allgemeinen die Dreifachbindung ausschliesslich zur trans-Doppelbindung reduziert. Die Reduktion wird zweckmässig in einem inerten organischen Lösungsmittel durchgeführt. Beispiele geeigneter Lösungsmittel sind die oben im Zusammenhang mit der Umsetzung der Verbindungen der Formel I angegebenen Lösungsmittel, insbesondere die dort als bevorzugt genannten. Vorzugsweise kann das Alkoholat ohne Isolierung im gleichen Lösungsmittel direkt mit einem Aluminiumhydrid der Formel III reduziert werden. Temperatur und Druck sind nicht kritisch. Da die Reduktion insbesondere im Falle der Alkoholate jedoch schon bei tiefen Temperaturen rasch abläuft, wird vorzugsweise bei etwa -50 °C bis Raumtemperatur, insbesondere bei etwa -20 °C bis etwa 0 °C gearbeitet. Bei der Reduktion des Alkohols der Formel II kann eine höhere Temperatur, beispielsweise Raumtemperatur von Vorteil sein.

Bevorzugte Reduktionsmittel der Formel III sind diejenigen, worin M Lithium oder Natrium bedeutet. Ferner steht x vorzugsweise für 1, 2 oder 3, insbesondere für 2. R bedeutet vorzugsweise eine Gruppe der Formel $C_nH_{2n+1}$-O-$C_mH_{2m}$-O-. m und n bezeichnen vorzugsweise je eine Zahl von 1 bis 3. Besonders

bevorzugtes Reduktionsmittel ist Natrium-dihydrido-bis(2-methoxyäthoxy)aluminat. Das Reduktionsmittel kann in etwa äquivalenten Mengen oder vorzugsweise im Ueberschuss verwendet werden. Bevorzugt ist eine Menge von mindestens etwa 1,2 Aequivalenten, beispielsweise etwa 1,28-1,5 Aequivalenten an Reduktionsmittel. Ein grösserer Ueberschuss stört jedoch nicht. Bei Umsetzung des Alkohols der Formel II wird vorzugsweise eine entsprechend höhere Menge eingesetzt, da das Reduktionsmittel teilweise zur Deprotonierung des Alkohols verbraucht wird.

Die Hydrolyse des intermediär gebildeten Aluminiumkomplexes kann in an sich bekannter Weise, beispielsweise mit Wasser, mit organischen oder anorganischen Säuren, wie p-Toluolsulfonsäure, verdünnte Schwefelsäure, verdünnte Salzsäure und dergleichen, oder vorzugsweise mit Laugen, wie Natronlauge oder Kalilauge erfolgen. Die Hydrolyse kann gewünschtenfalls so durchgeführt werden, dass gleichzeitig auch die gegebenenfalls vorhandenen Aethergruppen hydrolysiert werden. Die Bedingungen, unter denen die hier verwendeten Schutzgruppen erhalten bleiben bzw. abgespalten werden, sind dem Fachmann grundsätzlich bekannt. Im allgemeinen gilt jedoch, dass die Schutzgruppen unter neutralen oder basischen Bedingungen und zum Teil auch unter schwach sauren Bedingungen erhalten bleiben, während sie unter sauren, insbesondere unter stark sauren Bedingungen abgespalten werden. Temperatur und Druck sind nicht kritisch. Im allgemeinen wird jedoch bei Atmosphärendruck und Raumtemperatur oder tieferer Temperatur, vorzugsweise bei etwa 0 °C bis Raumtemperatur gearbeitet.

Die Verbindungen der Formel IV können nach bekannten Methoden in Zeaxanthin übergeführt werden. Gemäss einer bevorzugten Variante wird eine Verbindung der Formel IV mit Halogenwasserstoff und Triarylphosphin zum Phosphoniumsalz der allgemeinen Formel

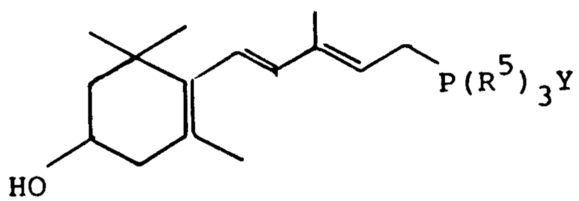

VI

worin $R^5$ Aryl und Y Halogen bedeuten,
umgesetzt und anschliessend das Phosphoniumsalz mit dem Dialdehyd der Formel

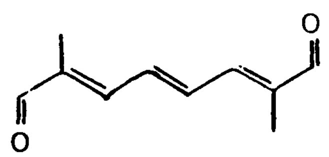

VII

zu Zeaxanthin kondensiert. Die Umsetzung kann nach den in Pure and Appl. Chem. 51, 535 (1979) und in J. Chem. Soc. C 404 (1971) beschriebenen Methoden erfolgen. Gegebenenfalls in Formel IV vorhandene Aetherschutzgruppen können vor oder während der Phosphoniumsalzbildung abgespalten werden. Die Reinigung erfolgt mit Vorteil durch Umkristallisation des Phosphoniumsalzes. Gewünschtenfalls kann der trans-Anteil durch thermische Isomerisierung, z.B. durch Erhitzen in Toluol, noch verbessert werden.

Die Verbindungen der Formeln I und II und die Alkoholate der Verbindungen der Formel II sind neu und ebenfalls Gegenstand der vorliegenden Erfindung.

Die Verbindungen der Formel I können nach dem in Schema 1 dargestellten Verfahren hergestellt werden, worin $R^1$ die obige Bedeutung hat, $R^6$ eine verätherte Hydroxygruppe, insbesondere eine der eingangs genannten verätherten Hydroxygruppen bezeichnet und $R^7$ eine Acyloxygruppe, insbesondere Acetoxy bedeutet.

Schema 1

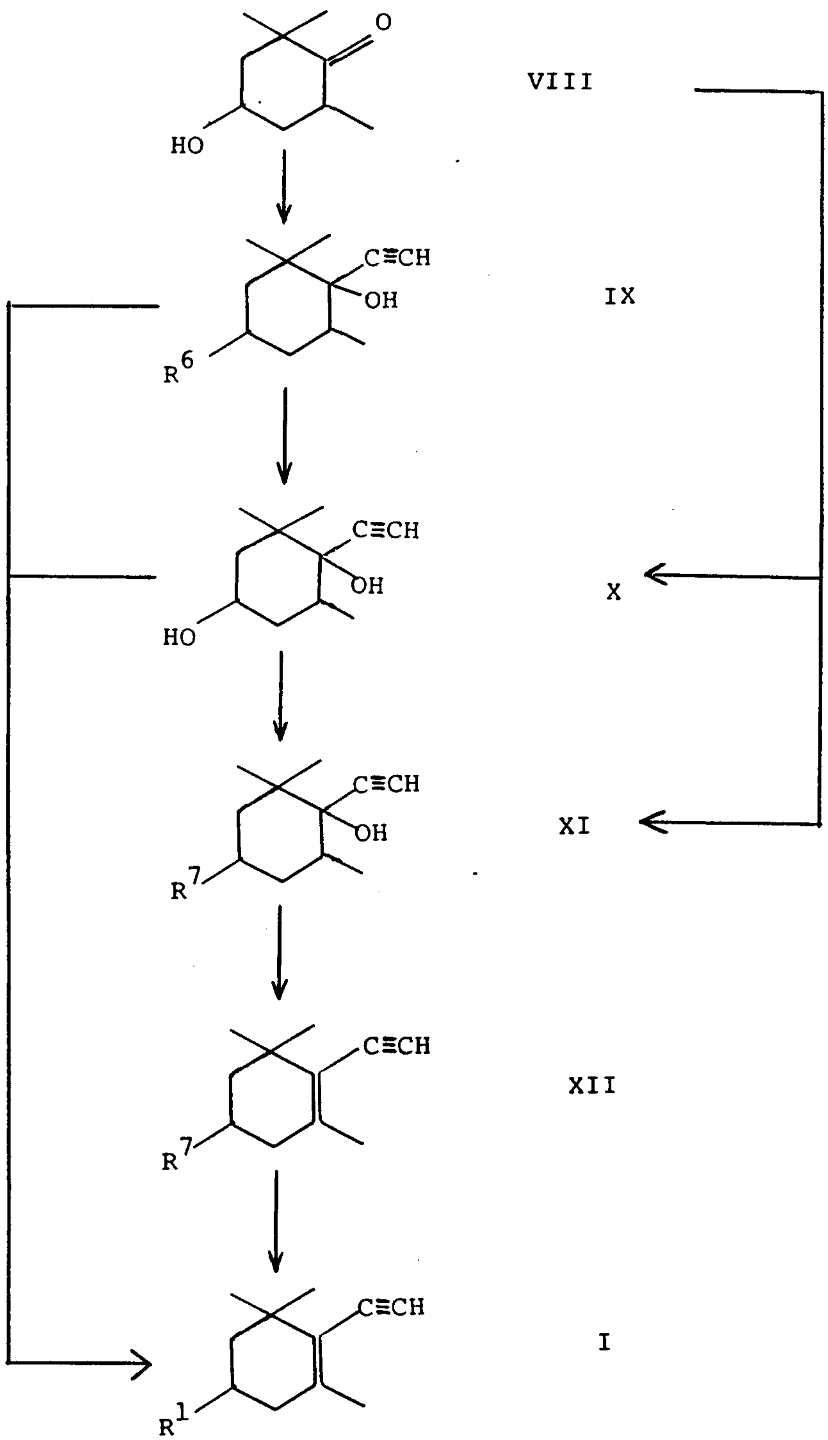

VIII

IX

X

XI

XII

I

Die Herstellung der Verbindungen der Formel I erfolgt gemäss Schema 1 aus der Verbindung der Formel VIII via Verbindungen der Formeln IX, X oder XI, welche in der allgemeinen Formel

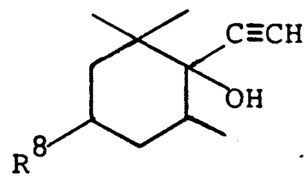

XIII

worin $R^8$ Hydroxy, eine verätherte Hydroxygruppe oder eine Acyloxygruppe bedeutet, zusammengefasst werden können.

Die Umsetzung der Verbindung der Formel VIII zu einer Verbindung der Formel IX erfolgt durch Verätherung der Hydroxygruppe und anschliessende Aethinylierung an der Ketogruppe. Geeignete Verätherungsmethoden, wie Umsetzung mit Alkylhalogenid, Aralkylhalogenid, Trialkylchlorisilan, Alkyl-1-alkenyläther, 3,4-Dihydro-2H-pyran und dergleichen sind dem Fachmann bekannt. Die Aethinylierung kann ebenfalls in an sich bekannter Weise erfolgen, beispielsweise mit Lithium-, Natrium- oder Kalziumacetylid in flüssigem Ammoniak oder vorzugsweise mit einem Lithiumacetylid-Ammoniak-Komplex in einem inerten organischen Lösungsmittel, z.B. Tetrahydrofuran oder Hexan. Das erhaltene Produkt weist vorwiegend die Hydroxygruppe in der gewünschten cis-Stellung zum vicinalen Wasserstoffatom auf.

Die Hydrolyse der Verbindungen der Formel IX zum Diol der Formel X kann in an sich bekannter Weise, z.B. mit organischen oder anorganischen Säuren wie Pyridinium-p-tosylat, p-Toluolsulfonsäure, Schwefelsäure und dergleichen, erfolgen.

Das Diol der Formel X kann auch direkt aus der Verbindung der Formel VIII erhalten werden durch Aethinylierung der Ketogruppe in analoger Weise zur oben beschriebenen Methode. Hierbei wird jedoch in der Regel ein cis/trans-Gemisch bezüglich der Stellung der tertiären Hydroxygruppe und des vicinalen Wasserstoffatoms erhalten.

Die Acylierung des Diols der Formel X zu einer Verbindung der Formel XI kann ebenfalls in an sich bekannter Weise durchgeführt werden, beispielsweise mit Acylanhydrid oder Acylhalogenid, und erfolgt selektiv an der sekundären Hydroxygruppe.

Die Verbindungen der Formel XI können auch direkt aus der Verbindung der Formel VIII erhalten werden durch Acylierung der Hydroxygruppe und anschliessende Aethinylierung an der Ketogruppe. Die Acylierung kann in an sich bekannter Weise erfolgen, beispielsweise mit Acylanhydrid oder Acylhalogenid. Die Aethinylierung kann ebenfalls in an sich bekannter Weise erfolgen, beispielsweise nach den bei der Herstellung der Verbindungen der Formel IX angegebenen Methoden. Hierbei wird ebenfalls die Hydroxygruppe vorwiegend in cis-Stellung relativ zum vicinalen Wasserstoffatom gebildet.

Die Dehydratisierung der Verbindungen der Formeln IX und X, welche direkt zu Verbindungen der Formel I führt, und die Dehydratisierung der Verbindungen der Formel XI werden vorzugsweise in Gegenwart von Kupfersulfat durchgeführt. Die Umsetzung erfolgt durch Erhitzen auf vorzugsweise mindestens etwa 140 °C und wird vorzugsweise in einem inerten Lösungsmittel mit einem Siedepunkt von mindestens etwa 140 °C, beispielsweise o-Xylol oder Silikonöl, durchgeführt. Das Kupfersulfat kann in katalytischen Mengen, vorzugsweise in etwa 1-20 Mol-% bezogen auf das Edukt, eingesetzt werden.

Die Verbindungen der Formel XII können in an sich bekannter Weise in Verbindungen der Formel I übergeführt werden durch Hydrolyse der Estergruppe $R^7$ und, gegebenenfalls, Verätherung der Hydroxygruppe. Die Hydrolyse der Estergruppe $R^7$ erfolgt vorzugsweise mit Laugen, wie Natronlauge oder Kalilauge, in einem Alkohol, wie Methanol oder Aethanol. Geeignete Verätherungsmethoden, wie Umsetzung mit Alkylhalogenid, Aralkylhalogenid, Trialkylchlorsilan, Alkyl-1-alkenyläther, 3,4-Dihydro-2H-pyran und dergleichen, sind dem Fachmann bestens bekannt.

Die Umsetzung via Verbindungen der Formeln XI und XII ergibt in der Regel höhere Ausbeuten als die direkte Dehydratisierung der Verbindungen der Formeln IX und X und ermöglicht zudem eine einfache Reinigung durch Destillation der Verbindung der Formel XII.

Die Herstellung der Verbindungen der Formel I erfolgt somit vorzugsweise durch Dehydratisierung einer Verbindung der Formel XIII in Gegenwart von Kupfersulfat, Hydrolyse einer gegebenenfalls vorhandenen Acyloxygruppe und, gegebenenfalls, Verätherung einer freien Hydroxygruppe. Die Verätherung kann vorzugsweise in situ vor der weiteren Umsetzung erfolgen.

Das vorliegende Verfahren ermöglicht die Herstellung von Zeaxanthin in Ausbeuten von über 70% bezogen auf die Verbindung der Formel VIII. Es eignet sich insbesondere auch zur Herstellung von natürlichem (3R,3′R)-Zeaxanthin. Vorzugsweise wird hierbei als Ausgangsmaterial der Formel VIII (4R,6R)-4-Hydroxy-2,2,6-trimethylcyclohexanon verwendet. Auf diesem Wege werden ausschliesslich Verbindungen der obigen Formeln erhalten, welche die gegebenenfalls geschützte, sekundäre Hydroxygruppe in R-Konfiguration und im Falle der Zwischenprodukte der Formel XIII die tertiäre Hydroxygruppe in S-Konfiguration und die Methylgruppe in R-Konfiguration aufweisen.

Das erfindungsgemässe Verfahren und die Herstellung der Ausgangsmaterialien werden durch die folgenden Beispiele weiter veranschaulicht.

Beispiel 1

a) 30 g (R)-4-Aethinyl-3,5,5-trimethyl-3-cyclohexan-1-ol wurden unter Argon in 200 ml absolutem Tetrahydrofuran gelöst. Die Lösung wurde mit 0,5 g Pyridinium-p-tosylat versetzt, dann bei 20-24 °C innert 10 Minuten mit 43,5 ml Isopropenylmethyläther versetzt und noch 1 Stunde bei Raumtemperatur gerührt.

b) Die erhaltene Lösung von (R)-4-Aethinyl-1-(1-methoxy-1-methyläthoxy)-3,5,5-trimethyl -3-cyclohexan wurde bei -10 °C bis -12 °C innert 10 Minuten tropfenweise mit 140 ml einer 1,56M Lösung von Butyllithium in Hexan versetzt und noch 10 Minuten bei dieser Temperatur gerührt. Dann wurde das Gemisch bei -10 °C innert 5 Minuten tropfenweise mit einer Lösung von 15,6 g Lithiumbromid in 150 ml absolutem Tetrahydrofuran versetzt und noch 15 Minuten gerührt. Anschliessend wurde die Reaktionslösung bei -12 °C bis -10 °C innert ca. 10 Minuten mit 22 ml Methylvinylketon versetzt und noch 30 Minuten gerührt.

c) Danach wurde die Reaktionslösung [enthaltend das Lithiumsalz von (R)-1-[4-(1-Methoxy-1-methyläthoxy)-2,6,6-trimethyl -1-cyclohexenyl]-3-methyl-4-penten-1-in-3-ol] bei -12 °C bis -8 °C innert 5 Minuten mit 63 ml einer 3,5M Lösung von Natrium-dihydro-bis(2-methoxyäthoxy)aluminat in Toluol versetzt. Die entstandene Suspension wurde 10 Minuten bei -10 °C und 40 Minuten bei 0 °C gerührt. Danach wurde die Suspension auf -5 °C abgekühlt, zuerst innert 10 Minuten mit einem Gemisch von 40 ml Aethanol und 60 ml Hexan und dann innert 5 Minuten mit 300 ml 28-prozentiger Natronlauge versetzt und noch 10 Minuten bei 0-5 °C kräftig gerührt. Anschliessend wurde das Reaktionsgemisch auf ein Gemisch von 600 ml 28-prozentiger Natronlauge und 1200 ml Hexan gegossen. Die wässrige Phase wurde abgetrennt und zweimal mit je 800 ml Hexan extrahiert. Die organischen Phasen wurden zweimal mit je 250 ml 28-prozentiger Natronlauge gewaschen, über Natriumsulfat getrocknet und filtriert. Das Filtrat wurde im Rotationsverdampfer am Wasserstrahlvakuum bei 40 °C Badtemperatur eingedampft und der ölige Rückstand 4 Stunden am Hochvakuum bei 60 °C getrocknet. Hierbei wurden 54,0 g rohes (R)-1-[4-(1-Methoxy-1-methyläthoxy)-2,6,6-trimethyl -1-cyclohexenyl]-3-methyl-1E,4-pentadien-3-ol als leicht gelbes Oel erhalten, welches ohne zusätzliche Reinigung weiter verwendet werden kann.

Beispiel 2

a) 56,6 g Triphenylphosphin wurden unter Argon in 175 ml Methanol suspendiert. Die Suspension wurde auf 0 °C abgekühlt, unter Rühren bei 0 °C innert 5 Minuten mit 19 ml 37-prozentiger Salzsäure versetzt und noch 10 Minuten bei 0 °C gerührt. Anschliessend wurde die Suspension bei 0 °C innert ca. 3 Stunden tropfenweise mit einer Lösung von 54,8 g rohem (R)-1-[4-(1-Methoxy-1-methyläthoxy)-2,6,6-trimethyl -1-cyclohexenyl]-3-methyl-1E,4-pentadien-3-ol (hersgestellt nach Beispiel 1) in 56 ml Methanol versetzt. Das Reaktionsgemisch wurde noch 30 Minuten bei 0 °C und 16 Stunden bei Raumtemperatur gerührt, dann mit 90 ml Wasser verdünnt und auf 200 ml Hexan gegossen. Die methanolische Phase wurde dreimal mit je 200 ml Hexan extrahiert, dann mit 150 ml Wasser und 7,5 g Aktivkohle versetzt, 20 Minuten bei Raumtemperatur gerührt und filtriert. Das Filtrat wurde im Rotationsverdampfer unter Wasserstrahlvakuum bei 40-45 °C Badtemperatur auf ein Volumen von 250-300 ml eingeengt (Entfernung des Methanols) und dann mit 250 ml Wasser und 90 ml gesättigter Kochsalzlösung versetzt. Das Gemisch wurde dreimal mit je 250 ml Methylenchlorid extrahiert. Die organischen Phasen wurden mit 205 ml Wasser und mit 90 ml gesättigter Kochsalzlösung gewaschen, über Natriumsulfat getrocknet und filtriert. Das Filtrat wurde im Rotationsverdampfer unter Wasserstrahlvakuum bei 40 °C Badtemperatur eingedampft. Der gelbliche Rückstand (116,5 g) wurde unter Argon bei 30-40 °C in 110 ml 1,2-Dichloräthan gelöst. Die Lösung wurde bei Raumtemperatur innert 4 Stunden mit 600 ml Aethylacetat versetzt. Die erhaltene Suspension wurde noch über Nacht bei Raumtemperatur weitergerührt, dann auf 0 °C abgekühlt und noch 30 Minuten bei 0 °C gerührt. Die Kristalle wurden abgenutscht, mit 300 ml kaltem Aethylacetat und mit 300 ml hexan gewaschen und dann 1 Stunde bei 45-50 °C am Wasserstrahlvakuum und über Nacht bei Raumtemperatur am Hochvakuum getrocknet. Hierbei wurden 81,97 g (R)-5-(4-Hydroxy-2,6,6-trimethyl-1-cyclohexenyl) -3-methyl-2,4-pentadienyl-triphenylphosphoniumchlorid [enthaltend 84,8% (2E,4E)- und 4,7% (2E,4Z)-Isomer] als weisse Kristalle mit Schmelzpunkt 193-195 °C erhalten. Dieses Material kann gewünschtenfalls direkt in der anschliessenden Wittig-Reaktion verwendet werden.

b) 43,54 g des erhaltenen Phosphoniumsalzes wurden unter Argon in 870 ml Toluol suspendiert. Die Suspension wurde unter intensivem Rühren 25 Minuten zum Rückfluss erhitzt, dann innert 30 Minuten auf ca. 60 °C abgekühlt und schliesslich mit einem Wasserbad auf Raumtemperatur abgekühlt. Die Kristalle wurden abgenutscht, dreimal mit je 150 ml Toluol und einmal mit 300 ml Hexan gewaschen und dann 1 Stunde bei 50 °C am Wasserstrahlvakuum und 20 Stunden bei Raumtemperatur am Hochvakuum getrocknet. Hierbei wurden 38,82 g (R)-5-(4-Hydroxy-2,6,6-trimethyl-1-cyclohexenyl) -3-methyl-2E,4E-pentadienyl-triphenylphosphoniumchlorid (enthaltend 1,3% 2E,4Z-Isomer) mit Schmelzpunkt 196-198 °C erhalten.

Beispiel 3

Ein Gemisch von 22 g (R)-5-(4-Hydroxy-2,6,6-trimethyl-1-cyclohexenyl) -3-methyl-2E,4E-pentadienyl-triphenylphosphoniumchlorid (hergestellt nach Beispiel 2), 3,28 g 2,7-Dimethyl-2,4,6-octatriendiol, 80 ml Aethanol und 16 ml 1,2-Butylenoxid wurde unter Rühren und Argonbegasung 20 Stunden zum Rückfluss erhitzt, dann auf -10 °C abgekühlt und 1 Stunde bei -10 °C gerührt. Das kristalline Produkt wurde abgenutscht, dreimal mit je 25 ml kaltem Aethanol gewaschen und über Nacht bei 80 °C am Hochvakuum getrocknet. Hierbei wurden 10,09 g rote Kristalle erhalten. Die Mutterlauge wurde im Rotationsverdampfer am Wasserstrahlvakuum bei 40 °C Badtemperatur eingeengt. Der Rückstand (16,7 g) wurde in 50 ml Aethanol und 5 ml 1,2-Butylenoxid gelöst und unter Rühren und Argonbegasung 20 Stunden zum Rückfluss erhitzt. Das Gemisch wurde auf -10 °C abgekühlt und 1 Stunde bei -10 °C gerührt. Die ausgefallenen Kristalle wurden abgenutscht, dreimal mit je 10 ml kaltem Aethanol gewaschen und zusammen mit dem Erstkristallisat unter Erwärmen und Argonbegasung in 400 ml Chloroform gelöst. Nach Abdestillation von ca. 250 ml Chloroform begann (3R,3′R)-Zeaxanthin teilweise zu kristallisieren. Innert 1,5 Stunden wurde das restliche Chloroform abdestilliert und simultan durch Zutropfen von 300 ml Aethanol ersetzt. Die Suspension wurde noch 1 Stunde unter Rückfluss gekocht, dann auf Raumtemperatur abgekühlt und noch 1 Stunde bei Raumtemperatur gerührt. Die Kristalle wurden abgenutscht, dreimal mit je 25 ml Aethanol gewaschen und zuerst bei 80 °C und dann am Hochvakuum bis zur Gewichstkonstanz getrocknet. Hierbei wurden 9,76 g (3R,3′R)-Zeaxanthin als dunkelrote Kristalle mit Schmelzpunkt 201-202 °C erhalten.

Beispiel 4

a) 10 g (R)-4-Aethinyl-3,5,5-trimethyl-3-cyclohexen-1-ol wurden unter Argon in 70 ml absolutem Tetrahydrofuran gelöst. Die Lösung wurde auf -20 °C abgekühlt, innert 5 Minuten mit 85,9 ml einer 1,56M Lösung von Butyllithium in Hexan versetzt und noch 10 Minuten bei -20 °C gerührt. Danach wurde das Gemisch bei -20 °C innert 2 Minuten mit einer Lösung von 5,3 g Lithiumbromid in 50 ml absolutem Tetrahydrofuran versetzt und noch 15 Minuten bei -20 °C gerührt. Anschliessend wurde das Reaktionsgemisch bei -20 °C innert 3 Minuten mit 9,92 ml Methylvinylketon versetzt und noch 1 Stunde bei -20 °C gerührt.

b) Danach wurde das Reaktionsgemisch [enthaltend das Dilithiumsalz von (R)-1-(4-Hydroxy-2,6,6-trimethyl-1-cyclohexenyl) -3-methyl-4-penten-1-in-3-ol] bei -20 °C innert 5 Minuten mit 34,8 ml einer 3,5M Lösung von Natrium-dihydrido-bis(2-methoxyäthoxy)aluminat in Toluol versetzt. Das Reaktionsgemisch wurde noch 2 Stunden bei 0 °C gerührt, anschliessend zuerst bei -20 °C innert 10 Minuten mit einem Gemisch von 15 ml Aethanol und 20 ml Hexan und dann bei 0 °C innert 10 Minuten mit 300 ml 28-prozentiger Natronlauge versetzt, und noch 5 Minuten gerührt. Danach wurde das Reaktionsgemisch auf 300 ml Hexan gegossen. Die wässrige Phase wurde abgetrennt und zweimal mit je 150 ml Hexan extrahiert. Die organischen Phasen wurden zweimal mit je 150 ml 28-prozentiger Natronlauge gewaschen, über Natriumsulfat getrocknet und filtriert. Das Filtrat wurde im Rotationsverdampfer am Wasserstrahlvakuum bei 40 °C Badtemperatur eingedampft und der Rückstand 2 Stunden bei Raumtemperatur am Hochvakuum getrocknet. Hierbei wurden 13,5 g rohes (R)-1-(4-Hydroxy-2,6,6-trimethyl-1-cyclohexenyl) -3-methyl-1E,4-pentadien-3-ol als gelbliches Oel erhalten. Chromatographische Reinigung an Kieselgel mit Hexan/Diäthyläther ergab 9,0 g Produkt als farbloses Oel.

Beispiel 5

a) 10 g (R)-4-Aethinyl-3,5,5-trimethyl-3-cyclohexen-1-ol wurden unter Argon in 30 ml Methylenchlorid gelöst. Die Lösung wurde auf 0 °C gekühlt und mit 12,7 ml Triäthylamin versetzt. Danach wurden bei 0 °C langsam 9,2 ml Trimethylchlorsilan zum Reaktionsgemisch zugetropft. Dann wurde das Reaktionsgemisch auf Raumtemperatur erwärmen gelassen und noch 1,5 Stunden bei Raumtemperatur gerührt.

Anschliessend wurde das Reaktionsgemisch auf 150 ml gesättigte Natriumhydrogencarbonat-Lösung gegossen und dreimal mit je 100 ml Methylenchlorid extrahiert. Die organischen Phasen wurden mit 100 ml halbgesättigter Natriumhydrogencarbonat-Lösung gewaschen, über Natriumsulfat getrocknet, genutscht und eingeengt. Hierbei wurden 14,24 g (R)-4-Aethinyl-3,5,5-trimethyl-1-trimethylsilyloxy -3-cyclohexen als violettes Oel erhalten.

b) 7,12 g (R)-4-Aethinyl-3,5,5-trimethyl-1-trimethylsilyloxy -3-cyclohexen wurden unter Argon in 35 ml Tetrahydrofuran gelöst. Die Lösung wurde auf -15° C abgekühlt, bei -15° C bis -10° C innert 5 Minuten tropfenweise mit 22,6 ml einer 1,56M Lösung von Butyllithium in Hexan versetzt und noch 5 Minuten bei -17° C gerührt. Dann wurde das Gemisch bei -17° C bis -9° C innert 5 Minuten tropfenweise mit einer Lösung von 2,5 g Lithiumbromid in 25 ml Tetrahydrofuran versetzt und noch 10 Minuten bei -20° C gerührt. Anschliessend wurde das Reaktionsgemisch bei -17° C innert 3 Minuten tropfenweise mit 3,6 ml Methylvinylketon versetzt und noch 35 Minuten bei -20° C gerührt.

c) Danach wurde das Reaktionsgemisch [enthaltend das Lithiumsalz von (R)-1-(2,6,6-Trimethyl-4-trimethylsilyloxy-1-cyclohexenyl) -3-methyl-4-penten-1-in-3-ol] bei -20° C bis -13° C innert 5 Minuten tropfenweise mit 10,2 ml einer 3,5M Lösung von Natrium-dihydrido-bis(2-methoxyäthoxy)aluminat in Toluol versetzt und noch 30 Minuten bei 0° C gerührt. Anschliessend wurde das Reaktionsgemisch tropfenweise zuerst mit einem Gemisch von 6,5 ml Aethanol und 10 ml Hexan und dann mit 50 ml 28-prozentiger Natronlauge versetzt. Nach Aufarbeitung in analoger Weise zu Beispiel 1c wurden 10,65 g rohes (R)-1-(2,6,6-Trimethyl-4-trimethylsilyloxy-1-cyclohexenyl) -3-methyl-1E,4-pentadien-3-ol als violettes Oel erhalten; Rf-Wert = 0,60 (Diisopropyläther).

d) 10,65 g rohes (R)-1-(2,6,6-Trimethyl-4-trimethylsilyloxy-1-cyclohexenyl) -3-methyl-1E,4-pentadien-3-ol wurden unter Stickstoff in 30 ml Tetrahydrofuran gelöst. Die Lösung wurde mit 13,8 g Tetrabutylammoniumfluorid-Trihydrat versetzt und noch 20 Minuten gerührt. Anschliessend wurde das Reaktionsgemisch auf 50 ml gesättigte Natriumhydrogencarbonat-Lösung gegossen und dreimal mit je 100 ml Aethylacetat extrahiert. Die organischen Phasen wurden über Natriumsulfat getrocknet, genutscht und eingeengt. Chromatographische Reinigung des erhaltenen braunen Oeles (9,8 g) mit Hexan/Diäthyläther an Kieselgel und anschliessende Trocknung am Hochvakuum ergab 6,02 g (R)-1-(4-Hydroxy-2,6,6-trimethyl-1-cyclohexenyl) -3-methyl-1E,4-pentadien-3-ol.

## Beispiel 6

a) 10 g (R)-4-Aethinyl-3,5,5-trimethyl-3-cyclohexen-1-ol wurden unter Argon in 100 ml absolutem Tetrahydrofuran gelöst. Die Lösung wurde mit 0,2 g Pyridinium-p-tosylat versetzt, dann bei 22-32° C innert 10 Minuten tropfenweise mit 15 ml Isopropenylmethyläther versetzt und noch 1 Stunde bei Raumtemperatur gerührt.

b) Die erhaltene Lösung von (R)-4-Aethinyl-1-(1-methoxy-1-methyläthoxy) -3,5,5-trimethylcyclohexen wurde bei -24° C bis -20° C innert 10 Minuten tropfenweise mit 50 ml einer 1,56M Lösung von Butyllithium in Hexan versetzt und noch 15 Minuten bei dieser Temperatur gerührt. Dann wurde das Gemisch bei -25° C bis -20° C innert 5 Minuten tropfenweise mit Lösung von 5,28 g Lithiumbromid in 100 ml absolutem Tetrahydrofuran und anschliessend innert 10 Minuten tropfenweise mit einer Lösung von 10 ml Methylvinylketon in 40 ml absolutem Tetrahydrofuran versetzt. Das Reaktionsgemisch wurde noch 45 Minuten bei -20° C gerührt, dann auf 200 ml Eiswasser gegossen, mit 20-prozentiger Kaliumhydrogensulfat-Lösung auf pH 3-4 gestellt und dreimal mit je 100 ml Diäthyläther extrahiert. Die organischen Phasen wurden mit 100 ml gesättigter Kochsalzlösung gewaschen, über Natriumsulfat getrocknet und eingeengt. Hierbei wurden 25,4 g Rohprodukt von (R)-1-[4-(1-Methoxy-1-methyläthoxy)-2,6,6-trimethyl -1-cyclohexenyl]-3-methyl-4-penten-1-in-3-ol als gelbes Oel erhalten.

c) Das erhaltene Rohprodukt wurde in 150 ml Tetrahydrofuran gelöst. Die Lösung wurde mit 3 ml Wasser und 0,3 g Pyridinium-p-tosylat versetzt und 45 Minuten bei Raumtemperatur gerührt. Anschliessend wurde das Reaktionsgemisch auf 200 ml gesättigte Natriumhydrogencarbonat-Lösung gegossen und zweimal mit je 200 ml Aethylacetat extrahiert. Die organischen Phasen wurden mit 100 ml gesättigter Kochsalzlösung gewaschen, über Natriumsulfat getrocknet und eingeengt. Das erhaltene gelbe Oel (23,4 g) wurde mit Hexan/Diäthyläther an Kieselgel chromatographisch gereinigt. Hierbei wurden 13,65 g (95,6%) (R)-1-(4-Hydroxy-2,6,6-trimethyl-1-cyclohexenyl)-3-methyl -4-penten-1-in-3-ol als weisse kristalline Masse erhalten.

## Beispiel 7

a) 300 g (4R, 6R)-4-Hydroxy-2,2,6-trimethylcyclohexanon wurden in 154,7 ml Pyridin gelöst. Die Lösung

wurde bei -3°C bis 0°C innert 10 Minuten tropfenweise mit 1,2 l Acetanhydrid versetzt und dann noch 4,25 Stunden bei Raumtemperatur gerührt. Anschliessend wurde das Reaktionsgemisch am Rotationsverdampfer bei 60°C/16 mbar eingeengt, wobei 394,7 g Rohprodukt als gelbes Oel erhalten wurden. Destillation des Rohproduktes ergab bei ca. 95°C/0,07 mbar 378,4 g (99,4%) (4R,6R)-4-Acetoxy-2,2,6-trimethylcyclohexanon als farbloses Oel.

b) In einem Sulfierkolben wurden 150 ml flüssiger Ammoniak vorgelegt und innert 15 Minuten bei -45°C bis -40°C portionenweise 2,8 g Lithiumdraht zugegeben. Das Gemisch wurde noch 30 Minuten bei -40°C gerührt. Dann wurden innert ca. 45 Minuten 60 l Acetylen (2,4 Mol) in das Gemisch eingeleitet, wobei nach Einleiten von ca. 16,5 l Acetylen ein Farbumschlag von blau auf weiss erfolgte. Anschliessend wurden 150 ml Tetrahydrofuran tropfenweise zum Reaktionsgemisch zugesetzt und unter leichtem Acetylenstrom Ammoniak aus dem Gemisch abdestilliert. Sobald die Innentemperatur 0°C erreichte, wurde die Acetylen-Zufuhr eingestellt. Das Reaktionsgemisch wurde auf -10°C abgekühlt, bei -15°C bis -10°C innert 15 Minuten tropfenweise mit einer Lösung von 39,65 g (4R,6R)-4-Acetoxy-2,2,6-trimethylcyclohexanon in 50 ml absolutem Tetrahydrofuran versetzt und noch 15 Minuten bei -15°C bis -10°C gerührt. Danach wurde das Reaktionsgemisch bei -10°C bis 0°C innert 10 Minuten mit 100 ml Eisessig versetzt. Anschliessend wurde das Gemisch bei -10°C bis 0°C mit 100 ml 2N Schwefelsäure und bei ca. 0°C mit 200 ml Wasser versetzt und dreimal mit je 200 ml Methylenchlorid extrahiert. Die organischen Phasen wurden einmal mit 200 ml halbgesättigter Kochsalzlösung und zweimal mit je 200 ml halbgesättigter Natriumhydrogencarbonat-Lösung gewaschen, über Natriumsulfat getrocknet und eingeengt. Hierbei wurden 48,4 g Rohprodukt als oranges Oel erhalten, welches gemäss Gaschromatographie 87,3% (1R,4S,5R)-4-Aethinyl-4-hydroxy -3,3,5-trimethylcyclohexylacetat und 8,8% (1S,4R,6R)-1-Aethinyl-2,2,6-trimethyl-1,4-cyclohexandiol enthielt.

Das erhaltene Rohprodukt kann nach der in Beispiel 10 beschriebenen Methode direkt weiter umgesetzt werden. Gewünschtenfalls kann das Rohprodukt vor der weiteren Umsetzung mit Acetanhydrid erwärmt werden zwecks Ueberführung des Diols in (1R,4S,5R)-4-Aethinyl-4-hydroxy -3,3,5-trimethylcyclohexylacetat.

Beispiel 8

a) 156,2 g (4R,6R)-4-Hydroxy-2,2,6-trimethylcyclohexanon wurden unter Argon in 250 ml absolutem Tetrahydrofuran gelöst. Die Lösung wurde zunächst mit 0,25 g Pyridinium-p-tosylat und dann bei einer Temperatur von 15-25°C innert ca. 20 Minuten mit 156,5 g Isopropenylmethyläther versetzt. Die erhaltene Reaktionslösung enthaltend (4R,6R)-4-(1-Methoxy-1-methyläthoxy) -2,2,6-trimethylcyclohexanon wurde noch 90 Minuten bei Raumtemperatur gerührt und dann direkt weiterverarbeitet.

b) In einem Sulfierkolben wurden 750 ml flüssiger Ammoniak vorgelegt und innert 40 Minuten bei einer Innentemperatur von ca. -40°C portionenweise 14 g Lithiumdraht zugesetzt. Das Gemisch wurde noch 30 Minuten bei -40°C gerührt. Dann wurden innert 2-3 Stunden 150 l Acetylen (6,1 Mol) in das Gemisch eingeleitet, wobei nach Einleiten von ca. 80 l Acetylen ein Farbumschlag von blau auf weiss erfolgte. Anschliessend wurden 750 ml absolutes Tetrahydrofuran zum Reaktionsgemisch zugesetzt und unter ständigem Einleiten von Acetylen Ammoniak innert ca. 1 Stunde aus dem Gemisch abdestilliert. Sobald die Innentemperatur ca. 0°C erreichte, wurde die Acetylen-Zufuhr eingestellt und das Reaktionsgemisch bei 0-2°C innert ca. 30 Minuten tropfenweise mit der in Absatz a hergestellten Lösung von (4R,6R)-4-(1-Methoxy-1-methyläthoxy) -2,2,6-trimethylcyclohexanon versetzt. Das Reaktionsgemisch wurde noch 30 Minuten bei 0-2°C weitergerührt, dann bei 2-20°C innert 10 Minuten mit 400 ml Wasser versetzt und bei Raumtemperatur über Nacht stehen gelassen. Anschliessend wurde das Reaktionsgemisch mit Hilfe von 700 ml Hexan in einen Scheidetrichter transferiert. Die wässrige Phase wurde abgetrennt und noch zweimal mit je 700 ml Hexan extrahiert. Die organischen Phasen wurden mit 700 ml halbgesättigter Ammoniumchloridlösung und mit 700 ml halbgesättigter Kochsalzlösung gewaschen und dann über 200 g Natriumsulfat getrocknet und filtriert. Das Filtrat wurde im Rotationsverdampfer unter Wasserstrahlvakuum bei 40°C eingedampft und der Rückstand 4 Stunden am Hochvakuum bei Raumtemperatur getrocknet. Hierbei wurden 260 g rohes (1S,4R,6R)-1-Aethinyl-4-(1-methoxy-1-methyläthoxy) -2,2,6-trimethyl-1-cyclohexanol als gelbe Kristalle erhalten.

c) 260 g rohes (1S, 4R, 6R)-1-Aethinyl-4-(1-methoxy-1-methyläthoxy) -2,2,6-trimethyl-1-cyclohexanol wurden unter Argon in 1300 ml Tetrahydrofuran und 52 ml Wasser gelöst. Die Lösung wurde bei Raumtemperatur mit 2,54 g Pyridinium-p-tosylat versetzt und 1 Stunde gerührt. Anschliessend wurde das Reaktiongemisch mit Hilfe von 700 ml Aethylacetat in einen Scheidetrichter transferiert und dann mit 500 ml gesättigter Natriumhydrogencarbonat-Lösung und mit 500 ml gesättigter Kochsalzlösung gewaschen. Die wässrigen Phasen wurden zweimal mit Aethylacetat nachextrahiert. Die organischen Phasen wurden über Natriumsulfat getrocknet und filtriert. Das Filtrat wurde im Rotationsverdampfer unter

Wasserstrahlvakuum bei 40°C eingeengt und der Rückstand 30 Minuten am Hochvakuum getrocknet. Hierbei wurden 201 g rohes (1S,4R,6R)-1-Aethinyl-2,2,6-trimethyl-1,4-cyclohexandiol als farblose Kristalle erhalten; Rf-Wert (Hexan/Diäthyläther 4:1) 0,25.

Beispiel 9

201 g rohes (1S,4R,6R)-1-Aethinyl-2,2,6-trimethyl-1,4-cyclohexandiol (hergestellt nach Beispiel 8) wurden in 380 ml Pyridin gelöst. Die Lösung wurde bei 0°C innert 20 Minuten mit 500 ml Acetanhydrid versetzt und dann noch 16 Stunden bei Raumtemperatur gerührt. Anschliessend wurde die Reaktionslösung im Rotationsverdampfer unter Wasserstrahlvakuum eingedampft. Das erhaltene gelbe Oel wurde in 1 l Methylenclorid gelöst und die Lösung nacheinander mit 500 ml 2N Salzsäure, mit 400 ml kalter, gesättigter Kochsalzlösung und mit 400 ml gesättigter Natriumhydrogencarbonat-Lösung gewaschen. Die Waschlösungen wurden zweimal mit je 200 ml Methylenchlorid nachextrahiert. Die organischen Phasen wurden über Natriumsulfat getrocknet und filtriert. Das Filtrat wurde im Rotationsverdampfer unter Wasserstrahlvakuum bei 40°C eingeengt und der Rückstand 3 Stunden bei Raumtemperatur am Hochvakuum getrocknet. Hierbei wurden 246,9 g rohes (1R,4S,5R)-4-Aethinyl-4-hydroxy -3,3,5-trimethylcyclohexylacetat als gelbbraune Kristalle erhalten; Rf-Wert (Diisopropyläther) 0,70.

Beispiel 10

In einem Sulfierkolben mit Wasserabscheider wurden unter Argon 121,6 g rohes (1R,4S,5R)-4-Aethinyl-4-hydroxy -3,3,5-trimethylcyclohexylacetat (hergestellt nach Beispiel 9) in 1 l o-Xylol gelöst. Das Gemisch wurde mit 7,9 g Kupfer-(II)-sulfat versetzt und 2 Stunden unter Rückfluss und Wasserabscheidung gekocht und intensiv gerührt. Anschliessend wurde das Reaktionsgemisch auf Raumtemperatur abgekühlt und das unlösliche Kupfersulfat abfiltriert (Nachwaschen mit 400 ml Hexan). Das Filtrat wurde mit 400 ml Wasser und mit 400 ml halbgesättigter Natriumhydrogencarbonat-Lösung gewaschen. Die Waschlösungen wurden zweimal mit je 300 ml Hexan nachextrahiert. Die organischen Phasen wurden über Natriumsulfat getrocknet und filtriert. Das Filtrat wurde im Rotationsverdampfer bei 80 Torr und 40-60°C Badtemperatur auf ein Volumen von ca. 200-300 ml eingeengt. Fraktionierte Destillation der zurückbleibenden Xylol-Lösung am Hochvakuum ergab bei ca. 83°C/0,19-0,09 Torr 96,5 g (R)-4-Aethinyl-3,5,5-trimethyl-3-cyclohexen-1-ylacetat (Reinheit 98,1%) als farbloses Oel; Siedepunkt 78-82°C/0,14 Torr. Die chemische Ausbeute bezogen auf (4R,6R)-4-Hydroxy-2,2,6-trimethylcyclohexanon beträgt 93%.

Beispiel 11

183,8 g (R)-4-Aethinyl-3,5,5-trimethyl-3-cyclohexen -1-ylacetat (hergestellt nach Beispiel 10) wurden in 1 l Methanol gelöst. Die Lösung wurde auf 0°C abgekühlt und innert 15 Minuten portionenweise mit 74,2 g Kaliumhydroxid so versetzt, dass die Temperatur 11°C nicht überstieg. Das Gemisch wurde noch 15 Minuten bei 4-8°C und dann 30 Minuten bei Raumtemperatur gerührt. Anschliessend wurde das Reaktionsgemisch auf 2 l Wasser und 26,5 g Eisessig gegossen. Das Gemisch wurde dreimal mit je 500 ml Methylenchlorid extrahiert. Die organischen Phasen wurden mit 500 ml gesättigter Kochsalzlösung und mit 600 ml gesättigter Natriumhydrogencarbonat-Lösung gewaschen, über Natriumsulfat getrocknet und filtriert. Das Filtrat wurde im Rotationsverdampfer unter Wasserstrahlvakuum bei 30°C Badtemperatur fast vollständig eingeengt. Das zurückbleibende Oel wurde in 300 ml Petan gelöst und die Lösung im Rotationsverdampfer unter Wasserstrahlvakuum bei 30°C Badtemperatur vollständig eingedampft. Das erhaltene Oel wurde 30 Minuten bei Raumtemperatur am Hochvakuum getrocknet, wobei spontane Kristallisation (stark exotherm) einsetzte. Hierbei wurden 146 g (R)-4-Aethinyl-3,5,5-trimethyl-3-cyclohexen-1-ol (Reinheit 98%, chemische Ausbeute 98,8%) als farblose Kristalle mit leicht violettem Farbton erhalten; Rf-Wert (Diisopropyläther) 0,40.

**Patentansprüche**

**1.** Verfahren zur Herstellung von Verbindungen der allgemeinen Formel

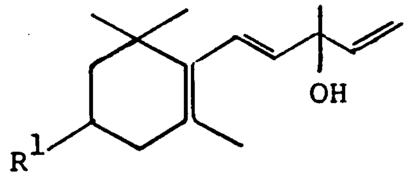

IV

worin $R^1$ Hydroxy oder eine verätherte Hydroxygruppe bedeutet,
und von Zeaxanthin, dadurch gekennzeichnet, dass man eine Verbindung der allgemeinen Formel

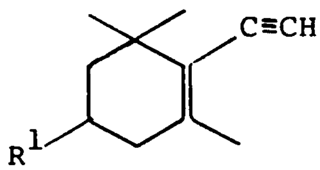

I

worin $R^1$ die obige Bedeutung hat,
in einem inerten organischen Lösungsmittel in das Acetylenid überführt und dieses mit Methylvinylketon umsetzt, das erhaltene Alkoholat oder den nach Hydrolyse des Alkoholates erhaltenen Alkohol der allgemeinen Formel

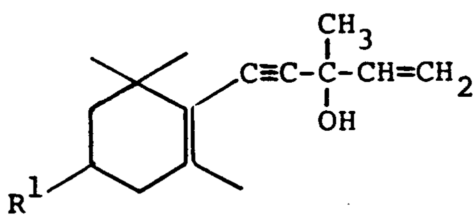

II

worin $R^1$ die obige Bedeutung hat,
in einem inerten organischen Lösungsmittel mit einem Aluminiumhydrid der allgemeinen Formel

$R_xMAlH_{4-x}$ III

worin M Alkalimetall bedeutet, R $C_1$-$C_{10}$-Alkoxy oder eine Gruppe der Formel $C_nH_{2n+1}$-O-$C_mH_{2m}$-O- darstellt, m und n unabhängig voneinander ganze Zahlen von 1 bis 7 bezeichnen und x für 0, 1, 2 oder 3 steht,
reduziert und anschliessend zur Verbindung der Formel IV hydrolysiert und dass man, gewünschtenfalls, die erhaltene Verbindung der Formel IV in Zeaxanthin überführt.

2. Verfahren nach Anspruch 1, dadurch gekennzeichnet, dass $R^1$ Trialkylsilyloxy oder eine Gruppe der allgemeinen Formel

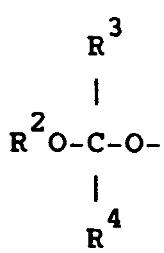

V

worin $R^2$ Alkyl bedeutet und $R^3$ und $R^4$ unabhängig voneinander Wasserstoff oder Alyl bedeuten, oder

$R^2$ und $R^3$ zusammen auch Tetramethylen bedeuten,
bezeichnet.

**3.** Verfahren nach Anspruch 1 oder 2, dadurch gekennzeichnet, dass man die Verbindung der Formel I mit einer Lithium-, Natrium- oder Magnesiumbase ins Acetylenid überführt.

**4.** Verfahren nach einem der Ansprüche 1 bis 3, dadurch gekennzeichnet, dass man die Umsetzung mit Methylvinylketon in Gegenwart eines Lithium- oder Cersalzes, vorzugsweise in Gegenwart von Lithiumbromid durchführt.

**5.** Verfahren nach einem der Ansprüche 1 bis 4, dadurch gekennzeichnet, dass man mindestens 1,3 Moläquivalente Methylvinylketon bezogen auf die Verbindung der Formel I einsetzt.

**6.** Verfahren nach einem der Ansprüche 1 bis 5, dadurch gekennzeichnet, dass man als inertes organisches Lösungsmittel einen Aether, vorzugsweise Tetrahydrofuran verwendet.

**7.** Verfahren nach einem der Ansprüche 1 bis 6, dadurch gekennzeichnet, dass man als Reduktionsmittel der Formel III Natrium-dihydrido-bis(2-methoxyäthoxy)aluminat einsetzt.

**8.** Verfahren nach einem der Ansprüche 1 bis 7, dadurch gekennzeichnet, dass man die R-Form der Verbindung der Formel I umsetzt.

**9.** Verfahren nach einem der Ansprüche 1 bis 8, dadurch gekennzeichnet, dass man eine erhaltene Verbindung der Formel IV mit Halogenwasserstoff und Triarylphosphin zu einem Phosphoniumsalz der allgemeinen Formel

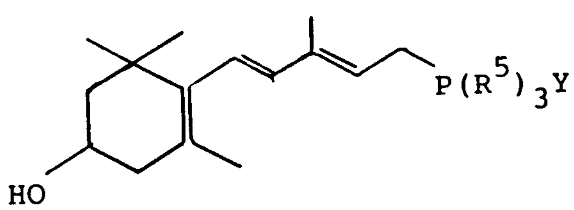

VI

worin $R^5$ Aryl und Y Halogen bedeuten,
umsetzt und das Phosphoniumsalz mit dem Dialdehyd der Formel

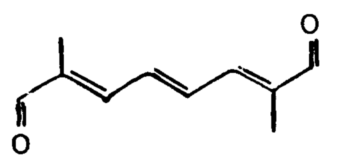

VII

zu Zeaxanthin kondensiert.

**10.** Verfahren nach einem der Ansprüche 1 bis 9, dadurch gekennzeichnet, dass man zur Herstellung der Verbindung der Formel I eine Verbindung der allgemeinen Formel

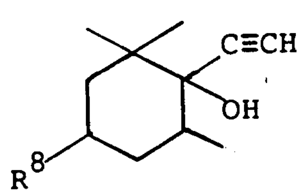

XIII

worin $R^8$ Hydroxy, eine verätherte Hydroxygruppe oder eine Acyloxygruppe bedeutet, in Gegenwart von Kupfersulfat dehydratisiert, eine gegebenenfalls vorhandene Acyloxygruppe hydrolysiert und, gegebenenfalls eine freie Hydroxygruppe veräthert.

**11.** Verbindungen der allgemeinen Formel

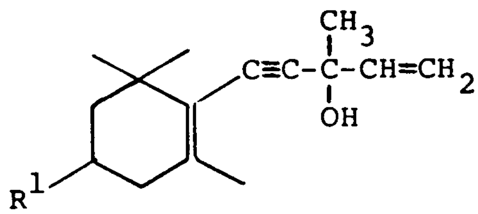

II

worin $R^1$ Hydroxy oder eine verätherte Hydroxygruppe bedeutet, und deren Alkoholate.

**12.** Verbindungen der Formel II nach Anspruch 11 und deren Lithium-, Natrium- und Magnesiumalkoholate.

**13.** Verbindungen der allgemeinen Formel

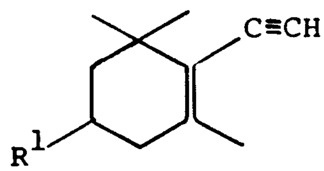

I

worin $R^1$ Hydroxy oder eine verätherte Hydroxygruppe bedeutet.

**Claims**

**1.** A process for the manufacture of compounds of the general formula

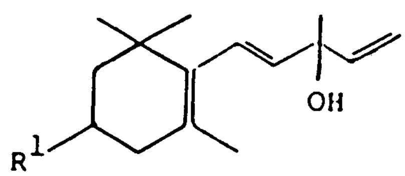

IV

wherein $R^1$ signifies hydroxy or an etherified hydroxy group, and of zeaxanthin, characterized by converting a compound of the general formula

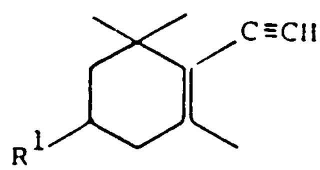

I

wherein $R^1$ has the above significance, in an inert organic solvent into the acetylenide and reacting this with methyl vinyl ketone, reducing the alcoholate obtained or the alcohol (obtained after hydrolysis of the alcoholate) of the general formula

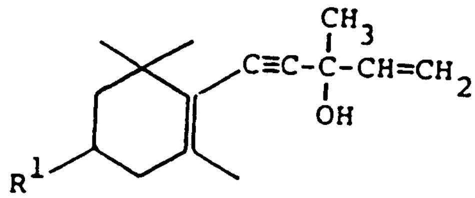

II

wherein $R^1$ has the above significance,
in an inert organic solvent with an aluminium hydride of the general formula

$R_xMAlH_{4-x}$ III

wherein M signifies alkali metal, R represents $C_1$-$C_{10}$-alkoxy or a group of the formula $C_nH_{2n+1}$-O-$C_mH_{2m}$-O-, m and n each individually denote whole numbers of 1 to 7 and x stands for 0, 1, 2 or 3,
and subsequently hydrolyzing to give the compound of formula IV and, if desired, converting the compound of formula IV obtained into zeaxanthin.

**2.** A process according to claim 1, characterized in that $R^1$ denotes trialkylsilyloxy or a group of the general formula

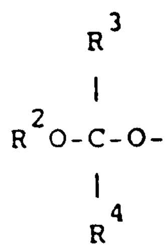

V

wherein $R^2$ signifies alkyl and $R^3$ and $R^4$ each independently signify hydrogen or alkyl or $R^2$ and $R^3$ together also signify tetramethylene.

**3.** A process according to claim 1 or 2, characterized in that the compound of formula I is converted into its acetylenide with a lithium, sodium or magnesium base.

**4.** A process according to any one of claims 1 to 3, characterized in that the reaction with methyl vinyl ketone is carried out in the presence of a lithium or cerium salt, preferably in the presence of lithium bromide.

**5.** A process according to any one of claims 1 to 4, characterized in that at least 1.3 mol equivalents of methyl vinyl ketone are used based on the compound of formula I.

**6.** A process according to any one of claims 1 to 5, characterized in that an ether, preferably tetrahydrofuran, is used as the inert organic solvent.

**7.** A process according to any one of claims 1 to 6, characterized in that sodium dihydrido-bis(2-methoxyethoxy)aluminate is used as the reducing agent of formula III.

**8.** A process according to any one of claims 1 to 7, characterized in that the R-form of the compound of formula I is reacted.

**9.** A process according to any one of claims 1 to 8, characterized in that a compound of formula IV obtained is reacted with a hydrogen halide and a triarylphosphine to give a phosphonium salt of the general formula

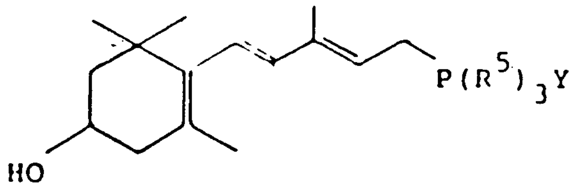

VI

wherein $R^5$ signifies aryl and Y signifies halogen, and the phosphonium salt is condensed with the dialdehyde of the formula

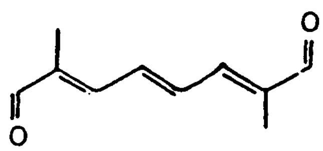

VII

to give zeaxanthin.

**10.** A process according to any one of claims 1 to 9, characterized in that, for the production of the compound of formula I, a compound of the general formula

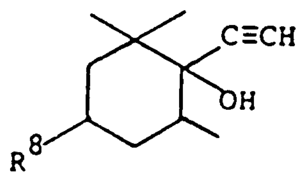

XIII

wherein $R^8$ signifies hydroxy, an etherified hydroxy group or an acyloxy group, is dehydrated in the presence of copper sulphate, an optionally present acyloxy group is hydrolyzed and, if desired, a free hydroxy group is etherified.

**11.** Compounds of the general formula

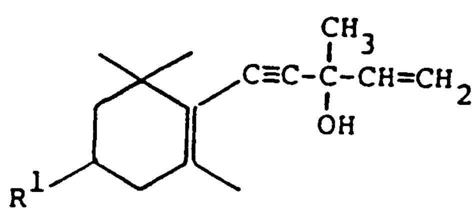

II

wherein $R^1$ signifies hydroxy or an etherified hydroxy group, and their alcoholates.

**12.** Compounds of formula II according to claim 11 and their lithium, sodium and magnesium alcoholates.

**13.** Compounds of the general formula

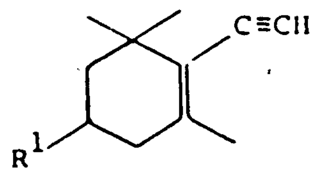

I

wherein $R^1$ signifies hydroxy or an etherified hydroxy group.

**Revendications**

**1.** Procédé de production de composés de formule générale :

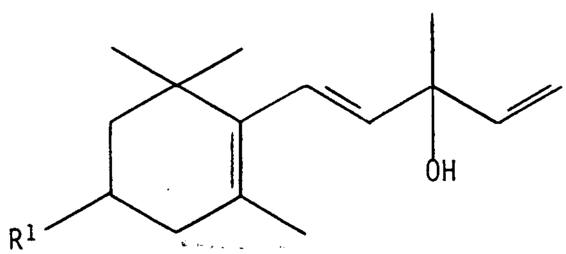

(IV)

dans laquelle $R^1$ est un hydroxy,
ou un groupe hydroxy éthérifié, et de zéaxanthine, caractérisé en ce qu'on transforme un composé de formule :

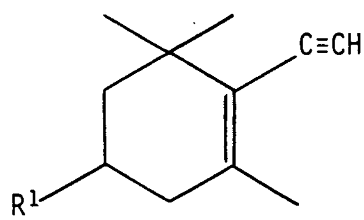

(I)

dans laquelle R a la signification donnée ci-dessus,
dans un solvant organique inerte en l'acétylure et on fait réagir celui-ci avec la méthylvinylcétone, on réduit l'alcoolate obtenu ou l'alcool obtenu après l'hydrolyse de l'alcoolate, de formule générale :

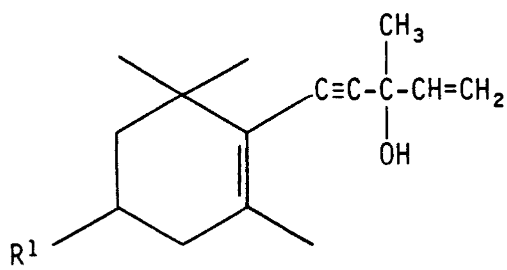

(II)

dans laquelle $R^1$ a la signification ci-dessus,
dans un solvant organique inerte avec un hydrure d'aluminium de formule générale :

$R_xMAlH_{4-x}$ (III)

dans laquelle M est un métal alcalin, R est un alcoxy en $C_1$-$C_{10}$ ou un groupe de formule $C_nH_{2n+1}$-O-

$C_mH_{2m}$-O-, m et n étant des nombres entiers indépendants l'un de l'autre compris entre 1 et 7, et x est 0, 1, 2 ou 3,
et ensuite on hydrolyse en le composé de formule IV, et en ce que, le cas échéant, on transforme le composé obtenu de formule IV en zéaxanthine.

**2.** Procédé selon la revendication 1, caractérisé en ce que $R^1$ représente un trialkylsilyloxy ou un groupe de formule générale :

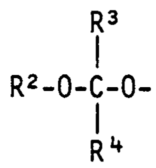

(V)

dans laquelle $R^2$ est un alkyle et $R^3$ et $R^4$ représentent indépendamment l'un de l'autre l'hydrogène ou un alkyle, ou $R^2$ et $R^3$ représentent aussi ensemble le tétraméthylène.

**3.** Procédé selon la revendication 1 ou 2, caractérisé en ce qu'on transforme le composé de formule I à l'aide d'une base de lithium, sodium ou magnésium en acétylure.

**4.** Procédé selon l'une des revendications 1 à 3, caractérisé en ce qu'on réalise la réaction avec la méthylvinylcétone en présence d'un sel de lithium ou de cérium, de préférence en présence de bromure de lithium.

**5.** Procédé selon l'une des revendications 1 à 4, caractérisé en ce qu'on utilise au moins 1,3 équivalent molaire de méthylvinylcétone par rapport au composé de formule I.

**6.** Procédé selon l'une des revendications 1 à 5, caractérisé en ce qu'on utilise comme solvant organique inerte un éther, de préférence le tétrahydrofurane.

**7.** Procédé salon l'une des revendications 1 à 6, caractérisé en ce qu'on utilise comme agent réducteur de formule III le dihydro-bis-(2-méthoxyéthoxy)-aluminate de sodium.

**8.** Procédé selon l'une des revendications 1 à 7, caractérisé en ce qu'on fait réagir la forme R du composé de formule I.

**9.** Procédé selon l'une des revendications 1 à 8, caractérisé en ce qu'on fait réagir un composé obtenu de formule IV avec un halogénure d'hydrogène et une triarylphosphine pour donner un sel de phosphonium de formule générale :

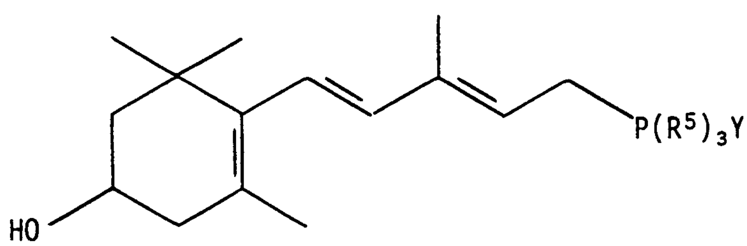

(VI)

dans laquelle $R^5$ est un aryle et Y un halogène,
et qu on condense le sel de phosphonium avec le dialdéhyde de formule :

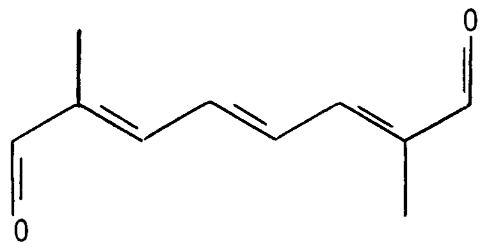

(VII)

en zéaxanthine.

**10.** Procédé selon l'une des revendications 1 à 9, caractérisé en ce que, pour préparer le composé de formule I, on déshydrate un composé de formule générale :

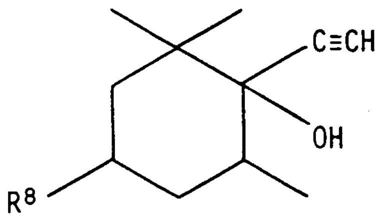

(XIII)

dans laquelle $R^8$ est un hydroxy, un groupe hydroxy éthérifié ou un groupe acyloxy, en présence de sulfate de cuivre, on hydrolyse un groupe acyloxy présent le cas échéant et, le cas échéant on éthérifie un groupe hydroxy libre.

**11.** Composés de formule générale :

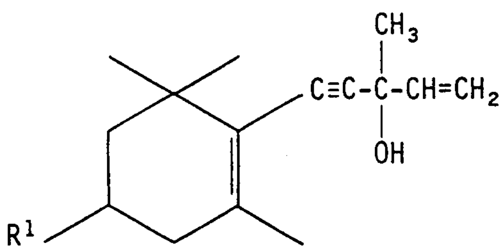

(II)

dans laquelle $R^1$ est un hydroxy ou un groupe hydroxy éthérifié, et leurs alcoolates.

**12.** Composés de formule II selon la revendication 11 et leurs alcoolates de lithium, sodium et magnésium.

**13.** Composés de formule générale :

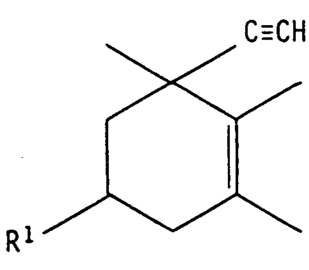

(I)

dans laquelle $R^1$ est un hydroxy ou un groupe hydroxy éthérifié.